# EUROPEAN PATENT SPECIFICATION

(11) **EP 4 297 670 B1**
(45) Date of publication and mention of the grant of the patent: **27.08.2025**
(21) Application number: 22705066.3
(22) Date of filing: 23.02.2022
(51) Int. Cl.: A61B 17/221, A61B 17/00, A61F 2/01, A61F 2/915

(54) **A SELF-EXPANDABLE MEDICAL DEVICE FOR ADVANCEMENT THROUGH VASCULATURE TO AN EXPANSION SITE IN A BLOOD VESSEL**
SELBSTEXPANDIERBARE MEDIZINISCHE VORRICHTUNG ZUM VORSCHIEBEN DURCH DAS GEFÄSSSYSTEM ZU EINER EXPANSIONSSTELLE IN EINEM BLUTGEFÄSS
DISPOSITIF MÉDICAL AUTO-EXPANSIBLE À AVANCER DANS LE SYSTÈME VASCULAIRE JUSQU'À UN SITE D'EXPANSION DANS UN VAISSEAU SANGUIN

(30) Priority: 26.02.2021 EP 21382165; 14.07.2021 WO PCT/EP2021/069623; 21.12.2021 EP 21383179
(43) Date of publication of application: 03.01.2024
(73) Proprietor: Anaconda BioMed, SL, 08192 Sant Quirze del Vallès Barcelona (ES)
(72) Inventor: GARCIA SABIDO, Daniel, 08720 Vilafranca del Penedès (ES); LIZARAZU GONZALEZ, Ane, 08013 Barcelona (ES); GALVE MURILLO, Iñaki, 08012 Barcelona (ES); ARAD HADAR, Ofir, 08197 Sant Cugat del Vallès (ES); NISSL, Thomas J.W., 37318 Mackenrode (DE); FERNÁNDEZ SÁNCHEZ, David, 08206 Sabadell (ES); JARA MUNS, Francesc, 08029 Barcelona (ES); PACE, Alizée, 1295 Mies, Vaud (CH)
(74) Representative: Torner, Juncosa I Associats, SL
(86) International application number: PCT/EP2022/054500
(87) International publication number: WO 2022/180083

(56) References cited:
- WO-A1-2021/016213
- US-A1- 2006 058 836
- US-A1- 2017 020 555
- US-A9- 2016 256 255
- US-A9- 2020 390 460

## Description

### Technical Field

The present invention is directed, in general, to the field of medical devices. In particular, the invention relates to a self-expandable medical device for advancement through vasculature to an expansion site in a blood vessel. The disclosure also provides methods of making and using such devices.

### Background of the Invention

A wide array of medical devices are inserted into the patient's body, advanced to an expansion site within the body in a contracted delivery configuration, and expanded to a deployed configuration to perform a therapy, a measurement, or another intervention at the expansion site. Many such devices are self-expandable so that they can expand to their deployed configurations when a constraining force is removed.

A variety of self-expandable medical devices may be compressed into a delivery configuration and placed within a catheter (e.g., guide catheter, microcatheter, delivery catheter etc.), and the catheter carrying the compressed device may be inserted through an entry site into the patient's vasculature and advanced to the desired site of an intervention or a measurement. Relative movement of the catheter with respect to the compressed device may place the device outside of the catheter, and the device may then self-expand at the site. Examples of such endovascularly delivered self-expandable devices include aspiration funnels, stents, and clot mobilizers.

In some cases, the self-expandable medical device is compressed and loaded into the distal end of the delivery catheter before insertion at the entry site into the patient's vasculature, and the delivery catheter and compressed device are advanced together through the vasculature to the device's expansion site. In some cases, the compressed device may be withdrawn a distance proximally within the delivery catheter during the catheter's advancement to make a portion of the delivery catheter distal to the compressed device more flexible to, e.g., enable the catheter to navigate tortuous vasculature. The compressed device is thereafter advanced to the distal end of the delivery catheter and/or the delivery catheter is retrieved proximally so that the compressed device can emerge and self-expand at the expansion site. The radially outward force exerted by the compressed self-expandable device on the inside wall of the catheter requires greater advancement and retraction forces than would be required for devices that are not self-expandable.

In other cases, the catheter or sheath may be advanced to the site of the intervention or measurement without the self-expandable medical device, and the device may thereafter be inserted into a proximal portion of the catheter and advanced through the catheter to the site of the intervention or measurement. Once again, the device's radially outward force against the inside wall of the catheter requires a greater advancement force than would be required for devices that are not self-expandable.

As discussed above, the compressed self-expandable medical device may have to be advanced through tortuous vasculature (e.g., the carotid syphon) to reach the device's expansion site. In some cases, the device may be advanced through the tortuous vasculature together with a delivery catheter into which the compressed device has been loaded. In other cases, the device may be advanced through the tortuous vasculature within a stationary delivery catheter. In either cases, the compressed device, or the combination of the compressed device and delivery catheter, should be sufficiently flexible to be advanceable through the tortuous vasculature with a minimum of advancement force.

In order for the incision at the vascular entry site to be as small as possible, the compressed self-expandable medical device and its delivery catheter should be as small as possible. Once at the expansion site, the device must expand from its compressed delivery configuration to its desired size and shape. In addition, some self-expandable medical devices (such as, e.g., clot mobilizers and aspiration funnels) must be able to apply a sufficient radially outward force when self-expanded at the expansion site. Expansion sites within the vasculature may vary in diameter; the self-expandable medical device must therefore be able to provide a sufficient radially outward force at every diameter within that range of diameters.

The prior art describes a variety of endovascularly delivered self-expandable medical devices.

For example, WO 03/075793 describes an endovascularly delivered self-expandable clot retriever. A generally tubular cage has a framework of struts that are biased to expand in an outward direction when unconstrained radially. The framework of the cage may be formed from wire segments, or it may be laser cut from a tube. The compressed cage is advanced to the desired expansion site within a microcatheter. Relative movement between a shaft extending proximally from the cage and the microcatheter allows the cage to emerge from the distal end of the microcatheter and self-expand. This publication does not disclose the radially outward forces exerted by the cage in its compressed and expanded configurations, nor does it describe structure enhancing the flexibility of the cage while preserving the cage's ability to navigate tortuous vasculature while compressed within the microcatheter and to exert desired radially outward forces in its compressed and expanded configurations.

US 2017/0020555 describes an endovascularly delivered reperfusion device with a scaffold that self-expands to compress and lyse a thrombus within a blood vessel. The scaffold is delivered in its compressed configuration within a microcatheter to the thrombus site. The scaffold self-expands when relative movement between the scaffold and microcatheter causes the scaffold to emerge from the microcatheter. A pusher at the proximal end of the scaffold may facilitate this relative movement. This publication discloses multiple scaffold patterns formed from laser cut tubes, including some patterns with features that enhance flexibility. It also discusses the general concept of providing a substantially constant radial outward force across a range of expansion diameters (corresponding to different blood vessel diameters), but without describing what aspects of the scaffold structure, or which of the disclosed patterns, provide the desired radial force characteristics.

US 9,700,331 describes a self-expandable vascular treatment device for capturing and removing embolic obstructions. This patent discloses multiple cell patterns for the expandable member. For example, it describes curvilinear cell patterns that enhance the flexibility of the compressed expandable member during delivery through tortuous vascular anatomy. It also discusses generally the desired range of expansion diameters and outward radial force applied by the expandable member over that range of expansion diameters. This patent does not describe, however, a cell structure that enhances flexibility of the expandable member in its compressed configuration while maintaining the desired outward radial characteristics of the expandable member over its range of expansion diameters.

WO 2019/055311 describes a medical device operable to extend and/or retract projections (e.g., anchors) in response to a stress applied by way of stretching and/or retracting the device. In some embodiments, the device is self-expandable and may be endovascularly delivered. This publication does not disclose the radially outward forces exerted by the cage in its compressed and expanded configurations, nor does it describe structure enhancing the flexibility of the cage while preserving the cage's ability to navigate tortuous vasculature while compressed within the microcatheter and to exert desired radially outward forces in its compressed and expanded configurations.

Therefore, new and improved self-expandable medical devices able to navigate and be advanced through vasculature, with proper pushability and flexibility, to be expanded in an expansion site in a blood vessel exerting appropriate outward radial forces, are therefore needed.

### Description of the Invention and additional embodiments of the disclosure

The invention is defined by the attached independent claims. Embodiments are described in the dependent claims. The methods disclosed are not claimed but are useful for understanding the invention.

According to one aspect, the present invention provides a self-expandable medical device for advancement through vasculature, of different sizes, shapes and dimensions, to an expansion site in a blood vessel. The self-expandable medical device comprises a working portion and a connection portion extending proximally from a proximal end of the working portion, the connection portion being adapted to be connected to a pusher for advancing the device through the vasculature to the expansion site. The working portion comprises a compressed configuration and an expanded configuration, a diameter of the expanded configuration being greater than a diameter of the compressed configuration, the working portion being configured to provide an outward radial force at every diameter between and including the diameter of the compressed configuration and the diameter of the expanded configuration, the working portion comprising a plurality of crowns of cells, each cell comprising an open area bordered by two proximal cell struts, two distal cell struts, and two middle cell struts, the proximal cell struts each extending distally from a common proximal end to a proximal end of a respective one of the two middle cell struts, the distal cell struts each extending proximally from a common distal end to a distal end of a respective one of the two middle cell struts, each middle cell strut in each crown bordering two adjacent cells in that crown, each of the middle cell struts being adapted and configured to be more flexible than the distal cell strut and proximal cell strut to which it extends.

According to another aspect, the present disclosure also provides a self-expandable medical device for advancement through vasculature, of different sizes, shapes and dimensions, to an expansion site in a blood vessel, comprising a working portion comprising a plurality of crowns of cells, each cell comprising an open area bordered by two proximal cell struts, two distal cell struts, and two middle cell struts, the middle cell struts bordering each cell having a width less than a width of the distal cell struts bordering that cell and less than a width of the proximal cell struts bordering that cell, the proximal cell struts each extending distally from a common proximal end to a proximal end of a respective one of the two middle cell struts, the distal cell struts each extending proximally from a common distal end to a distal end of a respective one of the two middle cell struts, each middle cell strut in each crown bordering two adjacent cells in that crown. The medical device also comprises a connection portion extending proximally from a proximal end of the working portion, the connection portion being adapted to be connected to a pusher for advancing the device through the vasculature to the expansion site.

The present teaching provides flexibility to the compressed configuration of the self-expandable medical device without compromising the outward radial force exerted by the device in its expanded configurations. This is particularly useful for self-expandable medical devices whose working portions are designed to apply a substantial outward radial force in their expanded configurations over a range of expansion diameters.

In an embodiment, the working portion also includes a tapered portion at the proximal end of the working portion. The tapered portion can comprise a plurality of tapered portion struts. The tapered portion has an expanded diameter at a proximal end that is smaller than an expanded diameter of the working portion.

In an embodiment, the tapered portion comprises at least one crown of cells of the plurality of crowns of cells.

According to the invention, the tapered portion has an expanded configuration defining a frustoconical shape with a closed structure.

According to the invention, the tapered portion further comprises a crown of cells with each cell comprising an open area bordered by two proximal cell struts and two distal cells struts, in each cell the proximal cell struts each extending distally from a common proximal end to proximal ends of the distal cell struts, the distal cell struts each extending distally from their proximal ends to a common distal end.

According to the invention, the tapered portion converges at a distal end of the connection portion.

According to the invention, the plurality of crowns of cells of the working portion defines a tubular-shaped section forming a cylindrically closed structure. Complementarily, the connection portion comprises a tubular-shaped section forming a cylindrically closed structure.

In an embodiment, the connection portion comprises at least one crown of cells, each cell comprising an open area bordered by two proximal cell struts, two distal cell struts, and two middle cell struts, the proximal cell struts each extending distally from a common proximal end to a proximal end of a respective one of the two middle cell struts, the distal cell struts each extending proximally from a common distal end to a distal end of a respective one of the two middle cell struts, each middle cell strut bordering two radially adjacent cells, each of the middle cell struts being adapted and configured to be more flexible than the distal cell strut and proximal cell strut to which it extends.

In some embodiments, the self-expandable medical device comprises a pusher extending proximally from the connection portion. In this case, particularly, the connection portion has a first attachment surface, and the pusher comprises a pusher connection portion extending distally, the pusher connection portion having a pusher attachment surface.

Moreover, in some embodiments, the pusher can comprise a tube, the pusher attachment surface comprising a distal portion of the tube attached to the first attachment surface of the connection portion. The tube can be a catheter or a hypotube. In other embodiments, the pusher can comprise a pusher wire, the pusher attachment surface comprising a distal portion of the pusher wire attached to the first attachment surface of the connection portion.

In some embodiments, the self-expandable medical device can comprise an improved attachment of its elements (i.e., self-expandable medical device and its pusher, e.g., a catheter). The device includes a catheter pusher comprising a catheter jacket, an inner catheter element surrounded by the catheter jacket, and a distal connection portion (i.e., pusher connection portion) comprising an extended portion of the inner catheter element extending distally beyond a distal end of the catheter jacket; the device (e.g., funnel or clot-mobilizer) attached to, and extending from, the distal connection portion of the catheter pusher, the device comprising a proximal connection portion; and an attachment between the device proximal connection portion and the pusher catheter distal connection portion adapted to keep the device connected to the catheter pusher, the attachment having an outer diameter substantially equal to an outer diameter of the pusher catheter.

The catheter jacket can be made of any suitable thermoplastic material or elastomeric/elastomer material, e.g., polyether block amide (PEBAX^{®}) with shore 25, 35, 40, 55, 63 or 72D. In a particular embodiment, the catheter jacket is made of a thermoplastic material, more particularly of PEBAX^{®} 40D.

In an embodiment, the inner catheter element comprises an inner liner disposed within the catheter jacket. In an embodiment, the inner liner comprises a polymer material, such as polytetrafluoroethylene (PTFE), among others.

In an embodiment, the inner catheter element further comprises a braided layer disposed within the catheter jacket. Particularly, the braided layer is concentrically disposed within the catheter jacket. The braided layer can be made of e.g., stainless steel, nitinol, liquid crystal polymer (LCP), etc. Particularly, it is made of nitinol.

In an embodiment, the attachment comprises a thermoplastic material extending between the proximal connection portion of the device and the distal connection portion of the catheter pusher. In another embodiment, the attachment comprises a thermoplastic material extending over the proximal connection portion of the device and over the distal connection portion of the catheter pusher. The thermoplastic material can extend between and/or over the proximal connection portion and the distal connection portion.

In the previous embodiments, the pusher can be attached to the proximal connection portion of the self-expandable medical device by a sleeve that is boned or mechanically held by a heat shrink process, by thermal melting or fusing, by means of a chemical process or by means of any other mechanical process.

In an embodiment, the middle cell struts bordering each cell have a width less than a width of the distal cell struts bordering that cell and less than a width of the proximal cell struts bordering that cell.

In an embodiment, the distal cell struts each have a width greater than or equal to 0.08 mm and less than or equal to 0.20 mm, in particular, in the range between 0.08 mm and 0.10 mm, for example 0.09 mm. In an embodiment, the proximal cell struts each have a width greater than or equal to 0.08 mm and less than or equal to 0.20 mm, in particular, in the range between 0.08 mm and 0.10 mm, for example 0.09 mm. In an embodiment, the middle cell struts each have a width greater than or equal to 0.05 mm and less than or equal to 0.15 mm, in particular, in the range between 0.06 mm and 0.10 mm, for example 0.075 mm.

The distal cell struts and the proximal cell struts in some embodiments have substantially the same width.

In an embodiment, the working portion comprises at least three crowns of cells. In other embodiments, the working portion comprises four, five, six (or even more) crowns of cells. In any of these embodiments, each crown of cells comprises at least four cells.

In an embodiment, each of the middle cell struts comprises a hinge portion. In another embodiment, each of the middle cell struts comprises two hinge portions. In any of these embodiments, each hinge portion of each middle cell strut is disposed between the proximal and the distal ends of the middle cell strut.

In an embodiment, the hinge portion comprises a bend that can be disposed either in a central portion of the middle cell strut or closer to one end of the middle cell strut than to another end of the middle cell strut. In other embodiments, the hinge portion comprises two bends.

In an embodiment, the hinge portion comprises a section of increased curvature.

In an embodiment, the hinge portion is integral with the middle cell strut.

In an embodiment, in each crown of cells of the working portion: the distal cell struts are disposed in a distal cell strut ring at equal distal cell strut positions along a longitudinal axis of the self-expandable medical device, the proximal cell struts are disposed in a proximal cell strut ring at equal proximal cell strut positions along the longitudinal axis of the device, and the common distal ends of the distal cell strut ring are circumferentially offset from the common proximal ends of the proximal cell strut ring.

In an embodiment, the common proximal ends of the distal cell strut ring of each crown of cells of the plurality of crowns of cells of the working portion are circumferentially offset from the common distal ends of the proximal cell strut ring of that crown of cells in one circumferential direction.

In an embodiment, the common proximal ends of the distal cell strut ring of a crown of cells of the plurality of crowns of cells of the working portion are offset from the common distal ends of the proximal cell strut ring of that crown of cells in a first circumferential direction and the common proximal ends of the distal cell strut ring of an adjacent crown of cells of the plurality of crowns of cells of the working portion are offset from the common distal ends of the proximal cell strut ring of that crown of cells in a second circumferential direction opposite to the first circumferential direction.

In some embodiments, the middle cell struts of a second crown of cells of the plurality of crowns of cells of the working portion extend distally from common distal ends of distal cell struts of a first crown of cells of the plurality of crowns of cells of the working portion to common proximal ends of proximal cell struts of a third crown of cells of the plurality of crowns of cells of the working portion, and the middle cell struts of the third crown of cells of the working portion extend distally from common distal ends of distal cell struts of the second crown of cells of the working portion to common proximal ends of proximal cell struts of a fourth crown of cells of the plurality of crowns of cells of the working portion. Note that the previous mention to "first", "second", "third" and "fourth" should be understood as reference elements and not to disposition elements (i.e., following a specific order). That is, in this particular embodiment, the indication to "first crown of cells" does not necessarily refer to the first crown from the proximal portion to the distal portion but a first reference element.

In some embodiments, the distal cell struts of each cell in each crown of cells can comprise the proximal cell struts of cells in a (posterior/subsequent) adjacent crown of cells.

Alternatively or complementary, in some embodiments, the proximal cell struts of each cell in each crown of cells can comprise the distal cell struts of cells in a (anterior/previous) adjacent crown of cells.

In an embodiment, the working portion further comprises at least one crown of intermediate cells. Each intermediate cell comprises an open area bordered by two proximal cell struts intersecting with two distal cell struts.

In an embodiment, the distal cell struts of each cell in the at least one crown of intermediate cells comprise the proximal cell struts of cells in an adjacent crown of cells.

In an embodiment, the proximal cell struts of each cell in the at least one crown of intermediate cells comprise the distal cell struts of cells in an adjacent crown of cells.

In an embodiment, the working portion is configured to expand from the compressed configuration to the expanded configuration with a diameter between 2.0 mm and 5.0 mm and to exert an outward radial force between 0 N and 3.5 N, for example between 0.5 N and 3.5 N. In an embodiment, the working portion is configured to expand from the compressed configuration to the expanded configuration with a diameter between 3.0 mm and 4.5 mm and to exert an outward radial force between 1 N and 2N.

In an embodiment, the working portion is configured to expand from the compressed configuration with a diameter being less than 2.0 mm to the expanded configuration with a diameter being at least 4.5 mm and to exert an outward radial force between 0.5 N and 3.5 N at every diameter between and including the diameter of the compressed configuration and the diameter of the expanded configuration.

In some embodiments, the self-expandable medical device comprises a funnel. In other embodiments, the self-expandable medical device comprises a clot mobilizer. In these other embodiments, the clot-mobilizer can comprise a stent or stent-retriever.

In some embodiments, the self-expandable medical device can include a coating. In an embodiment, the device comprises a coating on at least the tapered portion, or part thereof. In an embodiment, the device comprises a coating on the working portion. In an embodiment, the device comprises a coating on the connection portion. In yet another embodiment, the device comprises a coating on all or some of the tapered portion, working portion, and connection portion.

In the present invention, the diameter of the working portion in a maximum expanded configuration can be selected depending on the intended blood vessel diameter. Thus, in some embodiments, particularly if the self-expandable medical device comprises a clot mobilizer, such diameter can be equal to or greater than 2 mm and equal to or less than 6 mm. For the case of the self-expandable medical device comprising a funnel, such diameter can be equal to or greater than 4 mm and equal to or less than 8 mm, for example 5.2 mm or 6.6 mm.

In some embodiments, the self-expandable medical device can be made of a metal, a metal alloy or a composite including Nitinol or Nitinol/Platinum. In particular, the Nitinol material complies with the ASTM (American Society of Testing and Materials) F2063 (Standard Specification for Wrought Nickel-Titanium Shape Memory Alloys for Medical Devices and Surgical Implants). Nitinol is well known for applications in self-expanding structures, thanks to its shape-memory properties. However, other types of metals or even other types of materials can be also used, for example cobalt-chromium alloys or iron alloys such as stainless steel or spring steel; also, other materials with shape memory properties can be used, for example cooper or magnesium alloys. Thanks to the shape-memory properties and its structural configuration, the device is able to adapt its shape and its length to the surrounding lumen (e.g., surrounding carrier, catheter or vessel), also, while passing through the surrounding lumen, such that the device narrows and lengthens, allowing to retain any obstruction material inside.

In some embodiments, the self-expandable medical device can include radiopaque markers. In an embodiment, the radiopaque markers are included in the working portion. In other embodiments, the radiopaque markers are included in the connection portion. In yet another embodiment, the radiopaque markers are included in the attachment means of the device with the pusher.

In an embodiment, the self-expandable medical device is manufactured by providing a tube, having a longitudinal axis therethrough, providing a stationary source of laser radiation, generating a beam of laser radiation using the source of laser radiation, and cutting a desired pattern into the tube by scanning the beam over a desired region of the tube. In another embodiment, in which the self-expandable medical device is a clot mobilizer (e.g., a stent-retriever) it is manufactured by providing a wire; having a longitudinal axis therethrough, producing predetermined cuts of the cross section of the wire by means of an ultrashort pulse laser in order to produce a predetermined shape of the stent. For example, a suitable manufacturing method that could be used is described in US10434605B2.

Another aspect of the present disclosure is related to a thrombectomy apparatus including the self-expandable medical device of the previous aspects of the invention, and particular embodiments thereof.

Yet another aspect of the present disclosure, not claimed, provides a method of advancement of a self-expandable medical device through vasculature to an expansion site in a blood vessel of a patient. In some embodiments, the method includes the steps of advancing the self-expandable medical device through a delivery catheter in a delivery configuration to an expansion site within a blood vessel, the device including a working portion and a connection portion extending proximally from a proximal end of the working portion, the connection portion being adapted to be connected to a pusher for advancing the device through the vasculature to the expansion site, the working portion comprising a compressed configuration and an expanded configuration, a diameter of the expanded configuration being greater than a diameter of the compressed configuration, the working portion being configured to provide an outward radial force at every diameter between and including the diameter of the compressed configuration and the diameter of the expanded configuration, the working portion comprising a plurality of crowns of cells, each cell comprising an open area bordered by two proximal cell struts, two distal cell struts, and two middle cell struts, the proximal cell struts each extending distally from a common proximal end to a proximal end of a respective one of the two middle cell struts, the distal cell struts each extending proximally from a common distal end to a distal end of a respective one of the two middle cell struts, each middle cell strut in each crown bordering two adjacent cells in that crown, each of the middle cell struts being adapted and configured to be more flexible than the distal cell strut and proximal cell strut to which it extends; expanding the working portion into a deployment configuration (i.e., expanded configuration) in apposition with an inner wall of the blood vessel in the expansion site, with an outward radial force sufficient to reduce (i.e., partially or completely stop) proximal blood flow; and aspirating and capturing an obstructing material (e.g., a thrombus) into the device.

Some embodiments can include, after the expanding step, adapting the self-expandable medical device shape and length to the surrounding blood vessel. Some other embodiments can include the step of moving the device within the vasculature, the device lengthening as it narrows to adapt to the vessel diameter and to retain the obstructing material within the device. The self-expandable medical device can include a coating, in which case the method can include the step of permitting blood to flow through holes in the coating.

Some embodiments can include the step of, after the aspirating step, moving the device from the deployment configuration to a retrieval configuration (i.e., when the obstruction material is inside) to withdraw the device outside the patient body.

In some embodiments, the self-expandable medical device includes a pusher (e.g., a hypotube or a catheter) attached to a proximal end of the connection portion, in which case the aspirating step can also include the step of applying a vacuum through the catheter to interior spaces of the working portion.

Advantages of the self-expandable medical device derived from its structure and the parameters described herein, can be summarized as follows:
- Adequate radial force to be advanced in the compressed configuration and to be expanded in an expansion site. The device allows lower radial forces in small diameters and larger radial forces in large diameters, without, leaving a safety window. Consequently, the outward radial forces are maintained for a broad range of vessels with different diameters, better pushability is achieved, and the risk of damaging the vessel is reduced. Also, the tapered portion augments/enhances the outward radial force provided by the working portion.
- "Chinese finger trap" effect: this phenomenon occurs when an axial force is applied by pulling the device backwards or when the device contacts the vessel, so the device is retained or braked. If there is an object inside (e.g., a thrombus), the device tends to lengthen and therefore decreases its diameter, causing a compression which helps to retain the object inside the device.
- Pushability: when the mesh is compressed inside the carrier, the configuration of the plurality of crowns of cells allows a proper adaptability and movement when being compressed. Also, the tapered portion provide pushability to the device to be navigated through vasculature (e.g., the neuroanatomy) while minimizing buckling and kinking.
- Conformability inside the blood vessels, achieved mainly by the structure of the proximal, distal, and middle cell struts. These features allow the adaptation of the working portion to the curves of the vessels and avoid kinking of the device.

### Brief Description of the Drawings

The previous and other advantages and features will be more fully understood from the following detailed description of embodiments, with reference to the attached figures, which must be considered in an illustrative and non-limiting manner, in which:
Figs. 1A and 1B are 2D views showing two different embodiments of the proposed self-expandable medical device.
Figs. 2A and 2B are 3D views of the 2D embodiments of the proposed self-expandable medical device shown in Figs. 1A and 1B, respectively.
Figs. 3A and 3B are 3D views of the 2D embodiments of the proposed self-expandable medical device shown in Figs. 1A and 1B, respectively. In this case, unlike Figs. 2A and 2B, the self-expandable medical device comprises a coating.
Fig. 4 shows different examples of pusher elements that can be used for advancing the self-expandable medical device through the vasculature to the expansion site.
Figs. 5A and 5B illustrate other embodiments of the proposed self-expandable medical device.
Fig. 6A-6B schematically illustrates the geometry of the cross-sectional view of the first attachment surface and the pusher attachment surface, according to some embodiments.
Figs. 7A-7B schematically illustrate the geometry of the cross-sectional view of the first attachment surface and the pusher attachment surface, according to other embodiments.
Fig. 8 graphically illustrates a comparison (outward radial force vs. diameter) between different embodiments of the proposed self-expandable medical device, as explained in Example 1.

### Detailed Description of the Invention

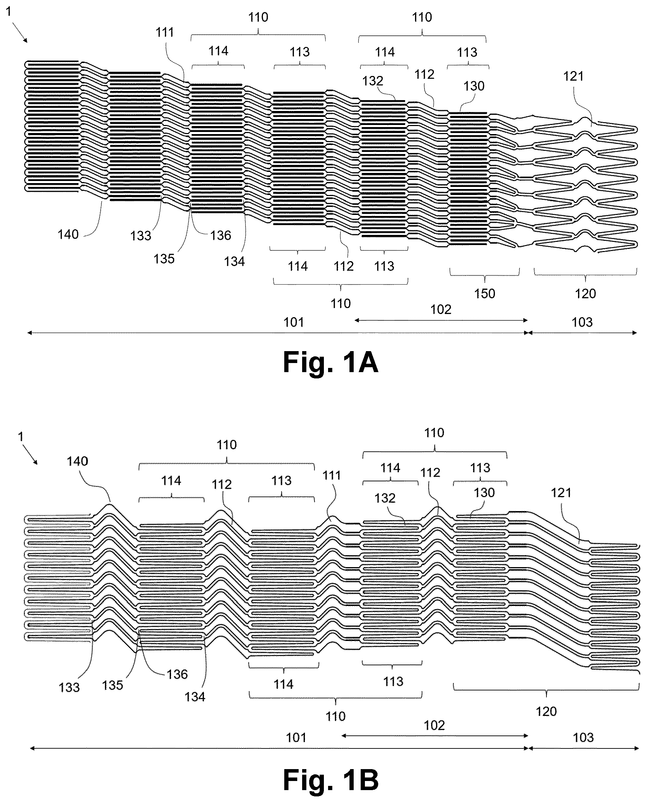

With reference to Figs. 1A and 1B (which shows the pattern to be cut from the solid tube to make the proposed self-expandable medical device 1, or simply device 1), therein two different embodiments of the device 1, for advancement through vasculature to an expansion site in a blood vessel are shown. In any of these embodiments, the device 1, which can be a funnel or a clot mobilizer, has a working portion 101 and a connection portion 103 that extends proximally from a proximal end of the working portion 101 and that can be connected to a pusher 200, 300, extending proximally from the connection portion.

The working portion 101 described therein is arranged and configured to provide an outward radial force at every diameter between and including a diameter of a compressed configuration of the working portion 101 and a diameter of an expanded configuration of the working portion 101. In some embodiments, the working portion 101 can expand from the compressed configuration with a diameter being less than 2.0 mm to the expanded configuration with a diameter being at least 4.0 mm and to exert an outward radial force between 0.5 N and 3.5 N at every diameter between and including the diameter of the compressed configuration and the diameter of the expanded configuration. In other embodiments, the working portion 101 can expand from the compressed configuration with a diameter being less than 3.0 mm to the expanded configuration a diameter being at least 4.5 mm and to exert an outward radial force between 1.0 N and 2.0 N at every diameter between and including the diameter of the compressed configuration and the diameter of the expanded configuration.

In the embodiments of Figs. 1A and 1B, the working portion 101 comprises a plurality of crowns 110 of cells 111 that form a tubular-shaped section forming a cylindrically closed structure. The connection portion 103, particularly, also has a tubular-shaped section forming a cylindrically closed structure. More in particular, in the embodiment of Fig. 1A, the working portion 101 has five crowns 110 of cells 111 whereas in the embodiment of Fig. 1B the working portion 101 has four crowns 110 of cells 111. This is not limitative, since in other embodiments, in this case not illustrated, the working portion 101 simply needs to comprise (at least) three crowns of cells, each having (at least) four cells.

Each of the cells of the working portion 101 has an open area bordered by two proximal cell struts 130, two distal cell struts 132 and two middle cell struts 112. The proximal cell struts 130 each extend distally from a common proximal end 134 to a proximal end 135 of a respective one of the two middle cell struts 112, and the distal cell struts 132 each extend proximally from a common distal end 136 to a distal end 133 of a respective one of the two middle cell struts 112. Each middle cell strut 112 in each crown border two adjacent cells 111 in that crown, and is adapted and configured to be more flexible than the distal cell strut 132 and proximal cell strut 130 to which it extends.

In some embodiments, the distal cell struts 132 of each cell 111 in each crown of cells can comprise the proximal cell struts 130 of cells 111 in a (posterior/subsequent) adjacent crown of cells. Alternatively or complementary, in some embodiments, the proximal cell struts 130 of each cell 111 in each crown of cells can comprise the distal cell struts 132 of cells 111 in a (anterior/previous) adjacent crown of cells.

In some embodiments, the middle cell struts 112 bordering each cell 111 can have a width less than a width of the distal cell struts 132 bordering that cell 111 and less than a width of the proximal cell struts 130 bordering that cell 111. More in particular, the middle cell struts 112 each can have a width greater than or equal to 0.05 mm and less than or equal to 0.15 mm, for example 0.075 mm, the distal cell struts 132 each can have a width greater than or equal to 0.08 mm and less than or equal to 0.20 mm, for example 0.09 mm, and the proximal cell struts 130 each can have a width greater than or equal to 0.08 mm and less than or equal to 0.20 mm, for example 0.09 mm. In yet some embodiments, the distal cell struts 132 and the proximal cell struts 130 can have substantially the same width.

As seen in the figures, the middle cell struts 112 integrate one or two hinge portions 140, having a section of increased curvature, that is/are arranged/disposed between the proximal and the distal ends of the middle cell strut 112. In Fig. 1B, each hinge portion 140 further involves a bend that can be either arranged in the central portion of the middle cell strut 112 or closer to one end of the middle cell strut 112 than to another end of the middle cell strut 112. In other embodiments, the hinge portion 140 comprises two bends.

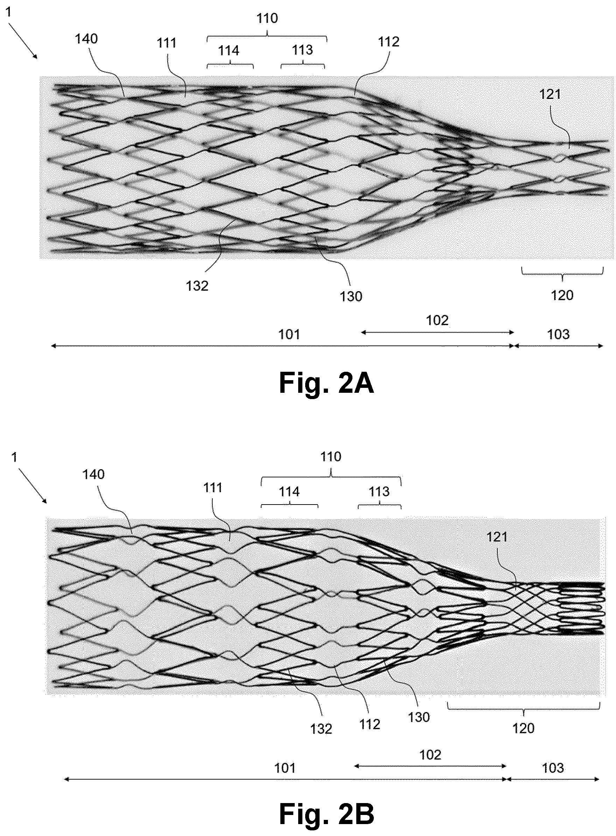

In each crown 110 of cells 111 of the working portion 101, the distal cell struts 132 are disposed in a distal cell strut ring 114 at equal distal cell strut positions along a longitudinal axis of the device 1; the proximal cell struts 130 are disposed in a proximal cell strut ring 113 at equal proximal cell strut positions along the longitudinal axis of the device 1; and the common distal ends of the distal cell strut ring 114 are circumferentially offset from the common proximal ends of the proximal cell strut ring 113, particularly, in one (see Fig. 1A or 2A) or more than one (see Figs. 1B or 2B) circumferential directions.

In some embodiments, the working portion 101 can also include at least one crown of intermediate cells, each intermediate cell comprising an open area bordered by two proximal cell struts intersecting with two distal cell struts. The distal cell struts of each cell in the at least one crown of intermediate cells can comprise the proximal cell struts of cells in an adjacent crown of cells. Similarly, the proximal cell struts of each cell in the at least one crown of intermediate cells can comprise the distal cell struts of cells in an adjacent crown of cells.

As can be seen in Figs. 1A and 1B, particularly, the working portion 101 also includes a tapered portion 102 at the proximal end thereof. The tapered portion 102 comprises a plurality of tapered portion struts and has an expanded diameter at a proximal end that is smaller than an expanded diameter of the working portion 101. In these embodiments, the tapered portion 102 has an expanded configuration that defines a frustoconical shape with a closed structure. Moreover, the tapered portion 102 includes one or more crowns of cells of the plurality of crowns 110 of cells 111. The tapered portion 102 converges at a distal end of the connection portion 103.

In the embodiment of Fig. 1A, the tapered portion 102 also includes a crown 150 of cells with each cell comprising an open area bordered by two proximal cell struts and two distal cells struts. In each cell, the proximal cell struts each extend distally from a common proximal end to proximal ends of the distal cell struts, the distal cell struts each extending distally from their proximal ends to a common distal end.

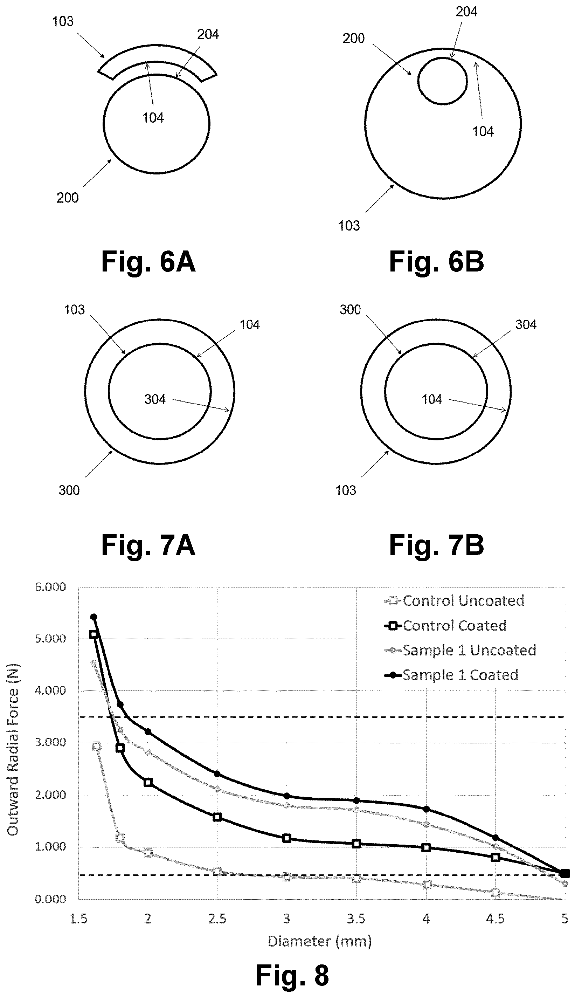

With regard to the connection portion 103, the latter comprises at least one crown 120 of cells 121, where each cell 121 has an open area bordered by two proximal cell struts, two distal cell struts, and two middle cell struts. The proximal cell struts each extend distally from a common proximal end to a proximal end of a respective one of the two middle cell struts. The distal cell struts each extend proximally from a common distal end to a distal end of a respective one of the two middle cell struts. Each middle cell strut border two radially adjacent cells, and is adapted and configured to be more flexible than the distal cell strut and proximal cell strut to which it extends. The connection portion 103 has an attachment surface 104 (or first attachment surface) to be connected to a pusher 200, 300 (see Figs. 6A-6B and 7A-7B for additional details thereof).

The device 1 can be made of a metal, a metal alloy or a composite including Nitinol or Nitinol/Platinum. However, other types of metals or even other types of materials can be also used, for example cobalt-chromium alloys or iron alloys such as stainless steel or spring steel; also, other materials with shape memory properties can be used, for example cooper or magnesium alloys.

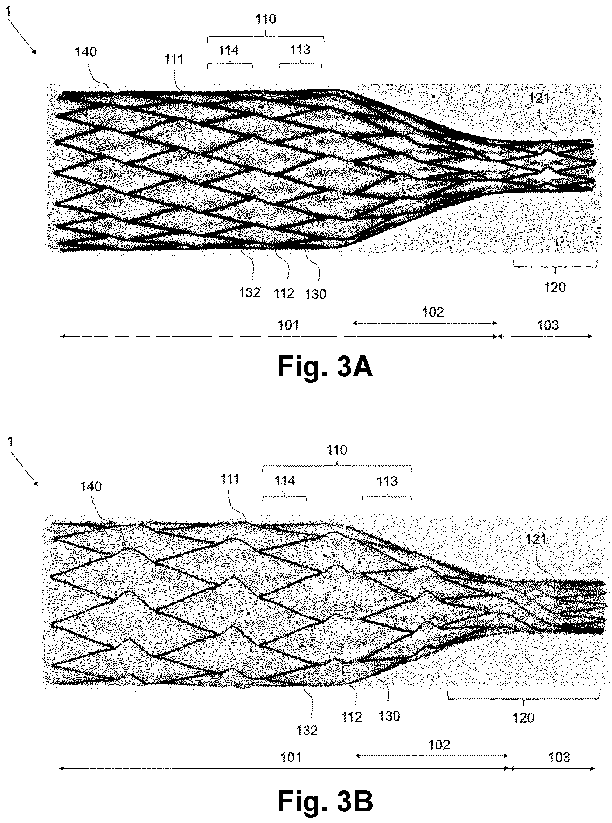

In some embodiments, as illustrated in Figs. 3A and 3B, the device 1 is fully coated, for example, with a polymer material. In some embodiments, the coating is a hydrophilic or a hydrophobic material. In some embodiments, the coating can be a non-permeable coating. Alternatively or complementarily, the coating can comprise an elastomeric or thermoplastic elastomer material such as polyurethane or silicone, among others. Although in the embodiments of Figs. 3A and 3B the coating is disposed over the entire surface of the device 1, in other embodiments, not illustrated, the coating can just cover at least one of the working portion 101, the tapered portion 102 or the connection portion 103.

The coating enables a safe retrieval/extraction of obstruction material, such as a thrombus, from a target site in a blood vessel, since the coating is atraumatic (i.e., avoids damaging the blood vessel wall). Further, the coating can increase the interaction of the device 1 with the obstruction material, for a proper extraction/retrieval. Also, the coating can increase the outward radial forces exerted by the device 1 to the surrounding blood vessel. Finally, a non-permeable covering at least in the tapered portion 102 allows the device 1 to reduce (i.e., partially, or completely stop) the blood flow.

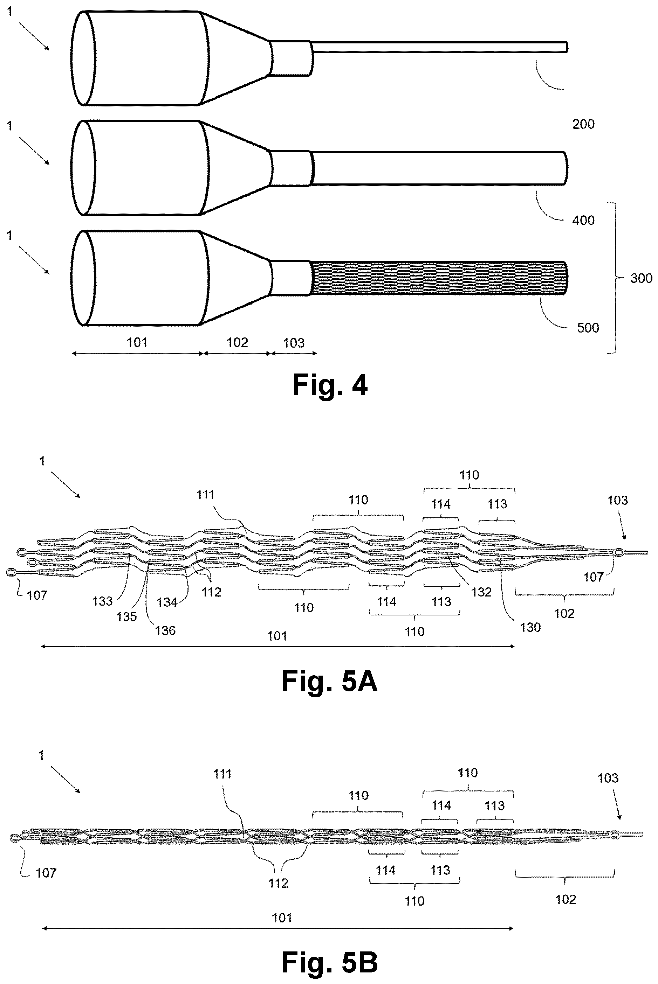

With reference now to Fig. 4, therein different pusher elements 200, 300 that can be attached to the connection portion 103 are shown. The pusher elements 200, 300 have a pusher connection portion that extends distally and a pusher attachment surface 204, 304 (see Figs. 6A-6B and 7A-7B for additional details thereof). As shown in Fig. 4, the pusher 300 includes, but is not limited to, either a catheter 400 or a hypotube 500. The pusher 200 can comprise a pusher wire (200).

With reference to Figs. 5A-5B, another embodiment of the device 1 is shown. In this case, different to the previous embodiments, although the working portion 101 and the tapered portion 102 are also cut from a tube, the connection portion 103 does not comprise a tubular-shaped section forming a cylindrically closed structure. As illustrated in the figures, the working portion 101 with crowns of cells 110 defined by proximal cell strut rings 113, distal cell strut rings 114, and middle cell struts 112 extend between common distal ends 136 of proximal cell struts 130 and common proximal ends 134 of distal cell struts 132. The distal cell struts 132 of a crown are disposed in a distal cell strut ring 114 at equal distal cell strut positions along a longitudinal axis of the device 1, and the proximal cell struts 130 of said crown 110 are disposed in a proximal cell strut ring 113 at equal positions along the longitudinal axis of the device 1.

Particularly, as shown in the flattened view of Fig. 5A (which shows the pattern to be cut from the solid tube to make the device 1), the middle cell struts 112 each have a width less than the width of the proximal cell struts 130 and the width of the distal cell struts 132, thereby making the middle cell struts 112 more flexible than the proximal cell struts 130 and the distal cell struts 132. In some embodiments, the distal cell struts 132 and the proximal cell struts 130 can have substantially the same width.

In the embodiment of Figs. 5A-5B the common proximal ends of the distal cell strut ring 114 of a first crown of cells of the plurality of crowns of cells (i.e., the proximal ends of the middle cell struts 112) are offset from the common distal ends of the proximal cell strut ring 113 of the first crown of cells (i.e., the distal ends of the middle cell struts 112) in a first circumferential direction, and the common proximal ends of the distal cell strut ring 114 of a second crown of cells of the plurality of crowns of cells (i.e., the proximal ends of the middle cell struts 112) are offset from the common distal ends of the proximal cell strut ring 113 of the second crown of cells (i.e., the distal ends of the middle cell struts 112) in a second circumferential direction opposite to the first circumferential direction. This pattern is repeated with subsequent crowns of cells, such that the common distal ends of proximal cell strut rings are alternately offset in the first circumferential direction and in the second circumferential direction in each successive crown of cells. Thus, the crowns are configured one to each other in a zig-zag pattern. In other embodiments (as seen in Fig. 1A), the crowns can be disposed only in one circumferential direction, being configured one to each other in a helix pattern.

The tapered portion 102 in the embodiment of Figs. 5A-5B comprises four tapered portion struts and a radiopaque marker 107. Additional radiopaque markers 107 are also disposed at the distal end of the working portion 101. Depending on the user needs, additional radiopaque markers 107 can be included in other sections of the device 1.

With reference to Figs. 6A-6B and Figs. 7A-7B, these figures illustrate multiple embodiments of the cross-sectional shape of the first attachment surface 104 of the connection portion 103 and the cross-sectional shape of the pusher attachment surface 204, 304 of the pusher 200, 300, and their correspondent disposition thereof.

Figs. 6A-6B show two embodiments in which the pusher 200 is a pusher wire . The pusher connection portion 204 and the first attachment surface 104 of the connection portion 103 are coaxially disposed. In Fig. 6A the connection portion 103 comprises the cross-sectional shape of a section of an annulus. In Fig. 6B the connection portion 103 comprises a circle cross-sectional shape.

Regarding now to Figs. 7A and 7B, in which the pusher 300 particularly comprises a tube. The tube can be a catheter 400 or an hypotube 500. Both figures show the coaxially disposition between a pusher connection portion 304 and the first attachment surface 104 of the connection portion 103. In Fig. 7A the pusher connection portion 304 is coaxially disposed over (i.e., coaxially outside) the attachment surface 104 of the connection portion 103. In Fig. 7B the attachment surface 104 of the connection portion 103 is coaxially disposed over (i.e., coaxially outside) the pusher connection portion 304.

### Example 1: Radial force comparison between the present self-expandable medical device and a control device

### Introduction

This test was developed in order to compare the outward radial forces (wall apposition) generated by the self-expandable medical device of the present invention (described in embodiments of Figs 1A, 2A -uncoated- and 3A -coated-) depending on the lumen diameter of the vessels. After extraction of the data, graphical description (outward radial force vs. vessel diameter), comparing a tested device (Sample 1, coated and uncoated) against a control device (Control, coated and uncoated), was generated.

### Methods

Both the Sample 1 and the Control were tested, with and without coating, for radial force (RF) measurement. The RX550 from Machine Solutions Inc. was used to measure both, the RFs and the diameter of the Sample 1 and of the Control. The specific geometry of the head (12 segments) allows compressing the tested device uniformly, with very low friction force. RX550 temperature was set to the defined test temperature. The samples were introduced in the head and profile was started: the RX bench decreases and/or increases the diameter according to specified profile and the RFs and diameter were recorded. At the end of the test, RX550 head was opened, and the tested device was retrieved from the RX550 then placed within its corresponding container. Lastly, Fig. 8 depicting the individual RF vs. diameter of the vessel was plotted.

The intended use for the tested self-expandable medical device is, particularly, between 2.0 mm and 5.0 mm. However, other self-expandable medical devices intended for larger vessel diameters (e.g., 6.0 mm) should follow the same requirements and therefore, present a flat-curve behavior, as explained below.

RF safety limits were defined starting from the vessel diameter of 2.0 mm, which should have a value lower than 3.5 N (i.e., upper safety limit), considering the value near 2 N ± 1.5 N for the Control. This value assured that there were not endothelial damage and, therefore, was set as the maximum value. On the other hand, the minimum value was defined ending in the vessel diameter of 5.0 mm, which should have a value greater than 0 N (lower safety limit), which was the minimum value to reach the vasculature target site and to extract a thrombus in optimal conditions, according to the Control, a value near 0.5 N is optimal. Most of neurovascular interventions are carried out in vessels with diameter in the range near and between 3.0 mm and 4.5 mm; therefore, a radial force value between 1 N and 2 N in those diameters is optimal to succeed in the intervention.

Two Controls were tested: one uncoated and another coated with a silicone polymer coating. Similarly, two Samples 1 were tested: one uncoated and another coated with a silicone polymer coating. The coating is atraumatic (i.e., reduces damaging the vessel), improves the lubricity of the device when being moved through vasculature or inside a carrier, and allows the device to reduce the blood flow in the expanded configuration. Furthermore, the addition of the coating increases the outward radial forces exerted by the device to the surrounding lumen (e.g., blood vessel or carrier).

### Results

As is shown in Fig. 8, the values of RF generated by the two tested devices (Samples 1), in relation to the diameter of the vessel, resulted between the maximum limit of 3.5 N and the minimum of 0.5 N, so the results were inscribed inside the optimal range of vessel diameters (from 2.0 mm to 5.0 mm), generating a flat curve for a broad range of vessels. Therefore, the curve provided by the proposed device 1 resulted in a manner such that allowed lower RFs (than the upper safety limit, i.e., 3.5 N) in small diameters and larger RFs (than the lower safety limit, i.e., greater than 0.5 N) in large diameters without, therefore, leaving the limited safety window. Remarkably, due to the fact of having RF values lower than the upper safety limit in small diameters, the risk of damage the vessel is reduced. Also, the outward radial force in the diameters inside the common range near 3.0 mm and 4.5 mm was inscribed inside the optimal range of 1-2 N, certifying the intervention success.

Therefore, the outward radial forces of the self-expandable medical device are maintained for a broad range of vessels with different diameters and the risk of damaging the vessel is reduced. In conclusion, the proposed medical device is usable in blood vessels of different diameters, and their flexibility and pushability certifies a proper navigability through vasculature to be expanded in a target location.

Throughout the description and claims the word "comprise" and its variations such as "comprising" are not intended to exclude other technical features, components, steps, operations and/or groups thereof. Additional objects, advantages will become apparent to those skilled in the art upon examination of the description or may be learned by practice of the invention. The foregoing description is provided primarily for exemplary purposes and should not be interpreted to limit the scope of the invention as it is set forth in the claims.

Additionally, as used herein, the singular forms "a", "an" and "the" are intended to include the plural forms as well, unless the context clearly indicates otherwise. Although the terms "first", "second". "third", "fourth" etc may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention. Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter. The phrase "about" or "approximately" may be used when describing magnitude and/or position to indicate that the value and/or position described is within a reasonable expected range of values and/or positions. For example, a numeric value may have a value that is +/-0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The present disclosure and/or some other examples have been described in the above.

According to descriptions above, various alterations may be achieved.

The scope of the present invention is defined in the following set of claims.

## Claims

1. A self-expandable medical device for advancement through vasculature to an expansion site in a blood vessel, comprising:
a working portion (101) having a compressed configuration and an expanded configuration, a diameter of the expanded configuration being greater than a diameter of the compressed configuration, the working portion (101) being configured to provide an outward radial force at every diameter between and including the diameter of the compressed configuration and the diameter of the expanded configuration, the working portion comprising a plurality of crowns (110) of cells (111), each cell comprising an open area bordered by two proximal cell struts (130), two distal cell struts (132), and two middle cell struts (112), the proximal cell struts (130) each extending distally from a common proximal end (134) to a proximal end (135) of a respective one of the two middle cell struts (112), the distal cell struts (132) each extending proximally from a common distal end (136) to a distal end (133) of a respective one of the two middle cell struts (112), each middle cell strut (112) in each crown bordering two adjacent cells (111) in that crown, each of the middle cell struts (112) being adapted and configured to be more flexible than the distal cell strut (132) and proximal cell strut (130) to which it extends, the working portion (101) further comprising a tapered portion (102) at the proximal end of the working portion (101); and
a connection portion (103) extending proximally from a proximal end of the working portion (101), the connection portion (103) being adapted to be connected to a pusher (200, 300) for advancing the device (1) through the vasculature to the expansion site,
wherein
the plurality of crowns (110) of cells (111) of the working portion (101) defines a tubular-shaped section forming a cylindrically closed structure;
the tapered portion (102) comprising a plurality of tapered portion struts;
**characterized in that** the tapered portion comprises at least one crown of cells of the plurality of crowns (110) of cells (111), has an expanded diameter at a proximal end that is smaller than an expanded diameter of the working portion (101) and an expanded configuration defining a frustoconical shape with a closed structure, and converges at a distal end of the connection portion (103); and
the connection portion (103) comprises a tubular-shaped section forming a cylindrically closed structure.

2. The device of claim 1, wherein the tapered portion (102) further comprises a crown (150) of cells with each cell comprising an open area bordered by two proximal cell struts (130) and two distal cells struts (132), in each cell the proximal cell struts (130) each extending distally from a common proximal end to proximal ends of the distal cell struts (132), the distal cell struts (132) each extending distally from their proximal ends to a common distal end.

3. The device of any one of claims 1-2, wherein the connection portion (103) comprises at least one crown (120) of cells (121), each cell (121) comprising an open area bordered by two proximal cell struts (130), two distal cell struts (132), and two middle cell struts (112), the proximal cell struts (130) each extending distally from a common proximal end to a proximal end of a respective one of the two middle cell struts (112), the distal cell struts (132) each extending proximally from a common distal end to a distal end of a respective one of the two middle cell struts (112), each middle cell strut (112) bordering two radially adjacent cells, each of the middle cell struts (112) being adapted and configured to be more flexible than the distal cell strut (132) and proximal cell strut (130) to which it extends.

4. The device of any one of claims 1-3, wherein the middle cell struts (112) bordering each cell have a width less than a width of the distal cell struts (132) bordering that cell and less than a width of the proximal cell struts (130) bordering that cell.

5. The device of any one of claims 1-4, wherein each of the middle cell struts (112) comprises one or two hinge portions (140), each hinge portion comprising one or two bends.

6. The device of claim 5, wherein each hinge portion (140) of each middle cell strut (112) is disposed between the proximal and the distal ends of the middle cell strut (112).

7. The device of any one of claims 1-6, further comprising a pusher (200, 300) extending proximally from the connection portion (103), the connection portion (103) having a first attachment surface (104), and the pusher (200, 300) comprising a pusher connection portion extending distally, the pusher connection portion having a pusher attachment surface (204, 304).

8. The device of claim 7, wherein the pusher (300) comprises a tube, the pusher attachment surface (304) comprising a distal portion of the tube attached to the first attachment surface (104).

9. The device of claim 8, wherein the tube is a catheter (400) or a hypotube (500).

10. The device of any one of claims 1-9, wherein the device (1) comprises a clot mobilizer or a funnel.

11. The device of claim 1, wherein the device is made of shape-memory materials such that is able to adapt its shape and its length to a surrounding lumen, also, while passing through the surrounding lumen, such that the device narrows and lengthens, allowing to retain obstruction material inside.

12. The device of claim 11, wherein the surrounding lumen is a carrier, a catheter or a vessel.

13. The device of claim 1, wherein the device is manufactured by providing a tube, having a longitudinal axis therethrough, providing a stationary source of laser radiation, generating a beam of laser radiation using the source of laser radiation, and cutting a desired pattern into the tube by scanning the beam over a desired region of the tube.

14. The device of claim 1, wherein the device comprises a coating on the tapered portion (102), or part thereof, on the working portion (101), or on the connection portion (103).

15. The device of claim 1, wherein the device comprises a coating on at least one of the working portion (101), the tapered portion (102), and the connection portion (103).

## Patentansprüche

1. Selbstexpandierbare medizinische Vorrichtung zum Vorschieben durch das Gefäßsystem zu einer Expansionsstelle in einem Blutgefäß, umfassend:
einen Funktionsteil (101) mit einer komprimierten Anordnung und einer expandierten Anordnung, wobei der Durchmesser der expandierten Anordnung größer ist als der Durchmesser der komprimierten Anordnung, wobei der Funktionsteil (101) so ausgelegt ist, dass er bei jedem Durchmesser zwischen dem Durchmesser der komprimierten Anordnung und dem Durchmesser der expandierten Anordnung, einschließlich desselben, eine nach außen gerichtete Radialkraft ausübt, wobei der Funktionsteil eine Vielzahl von Kronen (110) aus Zellen (111) umfasst, wobei jede Zelle einen offenen Bereich aufweist, der durch zwei proximale Zellstreben (130), zwei distale Zellstreben (132) und zwei mittlere Zellstreben (112) begrenzt ist, wobei sich die proximalen Zellstreben (130) jeweils distal von einem gemeinsamen proximalen Ende (134) bis zu dem proximalen Ende (135) der jeweils zugeordneten der beiden mittleren Zellstreben (112) erstrecken, wobei sich die distalen Zellstreben (132) jeweils proximal von einem gemeinsamen distalen Ende (136) bis zu dem distalen Ende (133) der jeweils zugeordneten der beiden mittleren Zellstreben (112) erstrecken, wobei jede mittlere Zellstrebe (112) in jeder Krone an zwei benachbarte Zellen (111) in dieser Krone angrenzt, wobei jede der mittleren Zellstreben (112) so ausgebildet und ausgelegt ist, dass sie flexibler ist als die distale Zellstrebe (132) und die proximale Zellstrebe (130), zu denen sie sich erstreckt, wobei der Funktionsteil (101) ferner einen Verjüngungsteil (102) am proximalen Ende des Funktionsteils (101) umfasst; und
einen Verbindungsteil (103), der sich proximal von einem proximalen Ende des Funktionsteils (101) erstreckt, wobei der Verbindungsteil (103) zur Verbindung mit einem Pushelement (200, 300) zum Vorschieben der Vorrichtung (1) durch das Gefäßsystem zur Expansionsstelle ausgelegt ist, wobei
die Vielzahl von Kronen (110) aus Zellen (111) des Funktionsteils (101) einen röhrenförmigen Abschnitt in Form einer zylindrisch geschlossenen Struktur aufweist;
wobei der Verjüngungsteil (102) eine Vielzahl von Verjüngungsteilstreben umfasst;
**dadurch gekennzeichnet, dass** der Verjüngungsteil mindestens eine Zellenkrone aus der Vielzahl von Kronen (110) aus Zellen (111) umfasst, am proximalen Ende einen gegenüber dem expandierten Durchmesser des Funktionsteils(101) kleineren expandierten Durchmesser aufweist, in expandierter Anordnung eine Kegelstumpfform mit einer geschlossenen Struktur bildet und am distalen Ende des Verbindungsteils (103) konvergiert; und
der Verbindungsteil (103) einen röhrenförmigen Abschnitt in Form einer zylindrisch geschlossenen Struktur aufweist.

2. Vorrichtung nach Anspruch 1, wobei der Verjüngungsteil (102) ferner eine Krone (150) aus Zellen umfasst, wobei jede Zelle einen offenen Bereich aufweist, der durch zwei proximale Zellstreben (130) und zwei distale Zellstreben (132) begrenzt ist, wobei sich in jeder Zelle die proximalen Zellstreben (130) jeweils distal von einem gemeinsamen proximalen Ende zu proximalen Enden der distalen Zellstreben (132) erstrecken, wobei sich die distalen Zellstreben (132) jeweils distal von ihren proximalen Enden bis zu einem gemeinsamen distalen Ende erstrecken.

3. Vorrichtung nach einem der Ansprüche 1 bis 2, wobei der Verbindungsteil (103) mindestens eine Krone (120) aus Zellen (121) umfasst, wobei jede Zelle (121) einen offenen Bereich aufweist, der durch zwei proximale Zellstreben (130), zwei distale Zellstreben (132) und zwei mittlere Zellstreben (112) begrenzt ist, wobei sich die proximalen Zellstreben (130) jeweils distal von einem gemeinsamen proximalen Ende bis zu dem proximalen Ende der jeweils zugeordneten der beiden mittleren Zellstreben (112) erstrecken, wobei sich die distalen Zellstreben (132) jeweils proximal von einem gemeinsamen distalen Ende bis zu dem distalen Ende der jeweils zugeordneten der beiden mittleren Zellstreben (112) erstrecken, wobei jede mittlere Zellstrebe (112) zwei radial benachbarte Zellen begrenzt, wobei jede der mittleren Zellstreben (112) so ausgebildet und ausgelegt ist, dass sie flexibler ist als die distale Zellstrebe (132) und die proximale Zellstrebe (130), zu denen sie sich erstreckt.

4. Vorrichtung nach einem der Ansprüche 1 bis 3, wobei die Breite der mittleren Zellstreben (112), die die einzelnen Zellen begrenzen, geringer ist als die Breite der distalen Zellstreben (132), die diese Zelle begrenzen, und geringer als die Breite der proximalen Zellstreben (130), die diese Zelle (111) begrenzen.

5. Vorrichtung nach einem der Ansprüche 1 bis 4, wobei jede der mittleren Zellstreben (112) einen oder zwei Gelenkabschnitte (140) umfasst, wobei jeder Gelenkabschnitt eine oder zwei Biegungen aufweist.

6. Vorrichtung nach Anspruch 5, wobei jeder Gelenkabschnitt (140) jeder mittleren Zellstrebe (112) zwischen dem proximalen und dem distalen Ende der mittleren Zellstrebe (112) angeordnet ist.

7. Vorrichtung nach einem der Ansprüche 1-6, die ferner ein sich proximal an den Verbindungsteil (103) anschließendes Pushelement (200, 300) umfasst, wobei der Verbindungsteil (103) eine erste Befestigungsfläche (104) aufweist, und das Pushelement (200, 300) einen sich distal anschließenden Pushelementverbindungsteil umfasst, wobei der Pushelementverbindungsteil eine Pushelementbefestigungsfläche (204, 304) aufweist.

8. Vorrichtung nach Anspruch 7, wobei das Pushelement (300) eine Röhre umfasst, wobei die Pushelementbefestigungsfläche (304) einen an der ersten Befestigungsfläche (104) befestigten distalen Abschnitt der Röhre umfasst.

9. Vorrichtung nach Anspruch 8, wobei die Röhre ein Katheter (400) oder ein Hypotube (500) ist.

10. Vorrichtung nach einem der Ansprüche 1 bis 9, wobei die Vorrichtung (1) einen Thrombus-Mobilisierer oder einen Trichter umfasst.

11. Vorrichtung nach Anspruch 1, wobei die Vorrichtung aus Formgedächtnismaterialien besteht und sich in Form und Länge an ein umgebendes Lumen anpassen kann, auch während der Passage durch das umgebende Lumen, wobei sich die Vorrichtung verengt und verlängert, wodurch Verschlussmaterial darin aufgenommen werden kann.

12. Vorrichtung nach Anspruch 11, wobei das umgebende Lumen ein Träger, ein Katheter oder ein Gefäß ist.

13. Vorrichtung nach Anspruch 1, wobei die Vorrichtung durch Bereitstellen einer Röhre mit Längsachse, Bereitstellen einer stationären Laserstrahlquelle, Erzeugen eines Laserstrahls mittels der Laserstrahlquelle und Schneiden eines gewünschten Musters in die Röhre durch Abtasten eines gewünschten Bereichs der Röhre mit dem Strahl hergestellt wird.

14. Vorrichtung nach Anspruch 1, wobei die Vorrichtung eine Beschichtung auf dem Verjüngungsteil (102), ganz oder teilweise, auf dem Funktionsteil (101) oder auf dem Verbindungsteil (103) aufweist.

15. Vorrichtung nach Anspruch 1, wobei die Vorrichtung eine Beschichtung auf mindestens einem der folgenden Elemente aufweist: dem Funktionsteil (101), dem Verjüngungsteil (102) und dem Verbindungsteil (103).

## Revendications

1. Dispositif médical auto-expansible destiné à être acheminé à travers le système vasculaire jusqu'à un site d'expansion dans un vaisseau sanguin, comprenant :
une partie active (101) ayant une configuration comprimée et une configuration expansée, un diamètre de la configuration expansée étant supérieur à un diamètre de la configuration comprimée, la partie active (101) étant conçue pour exercer une force radiale dirigée vers l'extérieur à chaque diamètre compris entre et incluant le diamètre de la configuration comprimée et le diamètre de la configuration expansée, la partie active comprenant une pluralité de couronnes (110) de cellules (111), chaque cellule comprenant une zone ouverte délimitée par deux montants proximaux de cellule (130), deux montants distaux de cellule (132) et deux montants centraux de cellule (112), les montants proximaux de cellule (130) s'étendant chacun distalement depuis une extrémité proximale commune (134) jusqu'à une extrémité proximale (135) de l'un des deux montants centraux de cellule (112), les montants distaux de cellule (132) s'étendant chacun proximalement depuis une extrémité distale commune (136) jusqu'à une extrémité distale (133) de l'un des deux montants centraux de cellule (112), chaque montant central de cellule (112) dans chaque couronne bordant deux cellules adjacentes (111) dans cette couronne, chacun des montants centraux de cellule (112) étant conçu et configuré pour être plus flexible que le montant distal de cellule (132) et le montant proximal de cellule (130) auxquels il est relié, la partie active (101) comprenant en outre une portion conique (102) à l'extrémité proximale de la partie active (101) ; et
une portion de connexion (103) s'étendant proximalement à partir d'une extrémité proximale de la partie active (101), la portion de connexion (103) étant conçue pour être reliée à un pousseur (200, 300) permettant d'acheminer le dispositif (1) à travers le système vasculaire jusqu'au site d'expansion,
dans laquelle :
la pluralité de couronnes (110) de cellules (111) de la partie active (101) définit une section en forme de tube formant une structure cylindrique fermée ;
la portion conique (102) comprenant une pluralité de montants de portion conique ;
**caractérisé en ce que** la portion conique comprend au moins une couronne de cellules de la pluralité de couronnes (110) de cellules (111), présente un diamètre expansé à une extrémité proximale qui est inférieur à un diamètre expansé de la partie active (101), et une configuration expansée définissant une forme tronconique avec une structure fermée, et converge à une extrémité distale de la portion de connexion (103) ; et
la portion de connexion (103) comprend une section en forme de tube formant une structure cylindrique fermée.

2. Le dispositif selon la revendication 1, dans lequel la portion conique (102) comprend en outre une couronne (150) de cellules, chaque cellule comprenant une zone ouverte délimitée par deux montants proximaux de cellule (130) et deux montants distaux de cellule (132), dans chaque cellule les montants proximaux de cellule (130) s'étendant chacun distalement depuis une extrémité proximale commune jusqu'aux extrémités proximales des montants distaux de cellule (132), les montants distaux de cellule (132) s'étendant chacun distalement depuis leurs extrémités proximales jusqu'à une extrémité distale commune.

3. Le dispositif selon l'une quelconque des revendications 1 à 2, dans lequel la portion de connexion (103) comprend au moins une couronne (120) de cellules (121), chaque cellule (121) comprenant une zone ouverte délimitée par deux montants proximaux de cellule (130), deux montants distaux de cellule (132) et deux montants centraux de cellule (112), les montants proximaux de cellule (130) s'étendant chacun distalement depuis une extrémité proximale commune jusqu'à une extrémité proximale de l'un des deux montants centraux de cellule (112), les montants distaux de cellule (132) s'étendant chacun proximalement depuis une extrémité distale commune jusqu'à une extrémité distale de l'un des deux montants centraux de cellule (112), chaque montant central de cellule (112) bordant deux cellules radialement adjacentes, chacun des montants centraux de cellule (112) étant conçu et configuré pour être plus flexible que le montant distal de cellule (132) et le montant proximal de cellule (130) auxquels il est relié.

4. Le dispositif selon l'une quelconque des revendications 1 ou 3, dans lequel les montants intermédiaires de cellule (112) bordant chaque cellule ont une largeur inférieure à celle des montants distaux de cellule (132) bordant cette cellule et inférieure à celle des montants proximaux de cellule (130) bordant cette cellule.

5. Le dispositif selon l'une quelconque des revendications 1 à 4, dans lequel chacun des montants centraux de cellule (112) comprend une ou deux portions articulées (140), chaque portion articulée comprenant un ou deux coudes.

6. Le dispositif selon la revendication 5, dans lequel chaque portion articulée (140) de chaque montant central de cellule (112) est disposée entre les extrémités proximale et distale du montant central de cellule (112).

7. Le dispositif selon l'une quelconque des revendications 1 à 6, comprenant en outre un pousseur (200, 300) s'étendant proximalement à partir de la portion de connexion (103), la portion de connexion (103) présentant une première surface de fixation (104), et le pousseur (200, 300) comprenant une portion de connexion de pousseur s'étendant distalement, ladite portion de connexion de pousseur présentant une surface de fixation de pousseur (204, 304).

8. Le dispositif selon la revendication 7, dans lequel le pousseur (300) comprend un tube, la surface de fixation de pousseur (304) comprenant une portion distale du tube fixée à la première surface de fixation (104).

9. Le dispositif selon la revendication 8, dans lequel le tube est un cathéter (400) ou un hypotube (500).

10. Le dispositif selon l'une quelconque des revendications 1 à 9, dans lequel le dispositif (1) comprend un mobilisateur de caillot ou un entonnoir.

11. Le dispositif selon la revendication 1, dans lequel le dispositif est fabriqué à partir de matériaux à mémoire de forme de sorte qu'il est apte à adapter sa forme et sa longueur à une lumière environnante, également lors de son passage à travers ladite lumière environnante, de sorte que le dispositif se rétrécit et s'allonge, permettant de retenir du matériau obstruant à l'intérieur.

12. Le dispositif selon la revendication 11, dans lequel la lumière environnante est un dispositif de transport, un cathéter ou un vaisseau.

13. Le dispositif selon la revendication 1, dans lequel le dispositif est fabriqué en fournissant un tube ayant un axe longitudinal, en fournissant une source stationnaire de rayonnement laser, en générant un faisceau de rayonnement laser à l'aide de ladite source, et en découpant un motif souhaité dans le tube en balayant le faisceau sur une région souhaitée du tube.

14. Le dispositif selon la revendication 1, dans lequel le dispositif comprend un revêtement sur la portion conique (102), ou une partie de celle-ci, sur la partie active (101), ou sur la portion de connexion (103).

15. Le dispositif selon la revendication 1, dans lequel le dispositif comprend un revêtement sur au moins l'une de la partie active (101), la portion conique (102), et la portion de connexion (103).
